# EUROPEAN PATENT APPLICATION

(11) **EP 4 768 603 A1**
(43) Date of publication of application: **01.07.2026**
(21) Application number: 24307290.7
(22) Date of filing: 24.12.2024
(51) Int. Cl.: C12Q 1/6886

(54) **METHOD OF DETECTING THE PRESENCE OF EXON 8 OF ANRIL IN A SUBJECT, KIT, SETS OF OLIGONUCLEOTIDES AND THEIR USES**

(71) Applicant: Université de Lorraine, 54000 Nancy (FR); Centre National de la Recherche Scientifique, 75016 Paris (FR)
(72) Inventor: MAENNER, Sylvain, 54000 NANCY (FR); AYADI, Lilia, 54000 NANCY (FR)
(74) Representative: Novagraaf Technologies

(57) **Abstract**

The present invention relates to a method of detecting the presence of exon 8 of ANRIL (Antisense Non-coding RNA in the INK4 Locus) in a subject, comprising the following steps of:
1) Preparation of total RNA from a biological sample of the subject,
2) Conversion of RNA molecules into complementary DNA,
3) Loop-mediated isothermal amplification using trans-exonic oligonucleotides that specifically produces an amplicon containing exon 8 fragment,
4) Addition of Cas12a/RNA guide complexes, wherein RNA guide specifically targets exon 8-containing fragment by base pairing,
5) Addition of a reporter DNA probe, and
6) Detection of cleavage products,
Wherein detection of cleavage products reveals the presence of exon 8 of ANRIL.

The present invention also relates to a method for in vitro predicting in vitro the efficacy of a therapy targeting exon 8 of ANRIL in a subject, to a kit for implementing the method and to a set of oligonucleotides.

## Description

### Technical field

The present invention refers to a method of detecting the presence of exon 8 of ANRIL (Antisense Non-coding RNA in the INK4 Locus) in a subject, to a kit and sets of oligonucleotides for implementing the method, and to use for predicting the efficacy of a therapy targeting exon 8 of ANRIL in a subject.

Therefore, the present invention has utility in medical fields.

In the description below, the references into brackets ([ ]) refer to the listing of references situated at the end of the text.

### Background of the Invention

In humans, multiple pathologies are due to inappropriate regulation of gene expression. The development of strategies aimed at restoring normal gene expression is therefore a major public health issue. These so-called "gene" therapies are complex and require several scientific obstacles to be overcome. One of them lies in the specificity of the therapeutic agent's action. Without specificity, the latter can cause a cascade of undesirable effects.

Non-coding RNAs do not have coding potential, i.e. they are not translated into proteins (e.g. ribosomal or transfer RNAs involved in the translation process of messenger RNAs).

Over the last decade, next generation sequencing approaches highlighted the unexpected diversity of RNAs lacking obvious protein-coding capacity (ncRNAs). The ncRNAs longer than 200-nts are named long non-coding RNAs (IncRNAs). Nowadays, more than 167,000 IncRNAs have been identified in human, many of them being involved in various critical processes including cell proliferation and cell differentiation. The deregulation of the expression of these IncRNAs can therefore affect cell homeostasis and consequently favour the occurrence and/or development of pathologies. They can then be qualified as pathogenic IncRNAs.

According to "the IncRNADisease database", IncRNAs are associated with 529 pathologies divided into several categories, including 3 major ones corresponding to cancers (44.2%), cardiovascular pathologies (11.6%) and neurodegenerative diseases (7.3%). Several IncRNAs are already used as biomarkers as their expression rate correlates with the diagnostic or even pronostic nature of certain pathologies. It is the case of IncRNA PCA3, used as a pronostic biomarker for prostate cancer.

Within the cell, IncRNAs can be located either in the cytoplasm or in the nucleus.

LncRNAs are key regulators of gene expression carrying both cytoplasmic and nuclear functions. Cytoplasmic IncRNAs mainly modulate gene expression by affecting mRNA stability or translation, while nuclear IncRNAs are mostly associated with the genome to regulate gene expression at the chromatin level. The latter implies the activities of epigenetic writers to targeted genomic loci, including for instance the Polycomb group proteins (PcG) composed by the Polycomb repressive complexes 1 and 2 (PRC1 and PRC2). These complexes are responsible for the conversion of euchromatin into heterochromatin by catalyzing the ubiquitylation of histone H2A on lysine 119 (H2AK119Ub) and the trimethylation of histone H3 on lysine 27 (H3K27me3), respectively. Interestingly, multiple IncRNAs have been shown to associate with the PcG and 20% of them are specific PRC2-binders in human cells.

In these gene regulatory mechanisms, nuclear IncRNAs must first recognize and specifically bind the DNA regions expression of which they regulate. To date, this step remains relatively undocumented. The two most widely described modes consist 1/ in the formation of a particular structure called R-loops involving the formation of canonical base pairs between the ncRNA and the DNA molecule and 2/ in the intervention of a DRBP (Doubled stranded RNA Binding Protein) capable of establishing a bridge between the DNA molecule and the IncRNA.

A third mode of interaction involves the formation of particular structures called triplexes [DNA/DNA:RNA]. These triplexes are non-canonical structures in which a single-stranded RNA (Triplex Forming Oligonucleotide, TFO) accommodates the major groove of the DNA double helix (Triplex Targeting Site, TTS). TTSs correspond exclusively to purine-rich sequences (Adenine-A/Guanine-G) and form non-canonical Hoogsteen or reverse Hoogsteen base pairs with TFO (Rajagopal P., and J. Feigon. "Triple-Strand Formation in the Homopurine:homopyrimidine DNA Oligonucleotides d(G-A)4 and d(T-C)4." Nature 339, no. 6226 (June 22, 1989): 637-40 ([1])). According to *in silico* analyses, millions of TTSs have been identified within the mammalian genome and located in regulatory regions such as promoters. The specificity and robustness of the TFO/TTS triplex correlate positively with the GC percentage of TFO and the number of Hoogsteen and inverse Hoogsteen base pairs forming between TFO and TTS (Maldonado et al.: "Purine- and Pyrimidine-Triple-Helix-Forming Oligonucleotides Recognize Qualitatively Different Target Sites at the Ribosomal DNA Locus." RNA (New York, N.Y.) 24, no. 3 (2018): 371-80 ([2])). Historically, triplexes [DNA/DNA:RNA] have been functionally associated with events of transcription, gene silencing and conversion, cell proliferation and double-stranded DNA breaks. Interestingly, several studies suggest that these structures may be also widely used by IncRNAs. Indeed, the formation of triplexes would allow IncRNAs to anchor themselves to chromatin and recruit protein complexes at the gene regions the expression of which they regulate.

ANRIL is one of the IncRNAs associated with PcG activities and several pathologies. It is transcribed from the *9p21* locus in the opposite direction to the *CDKN2A* and *CDKN28* (cyclin dependent kinase inhibitors 2A and 2B) genes. ANRIL promotes *in cis* the transcriptional silencing of the *CDKN2A* and *B* genes by recruiting the PcG to the *9p21* locus resulting in an increased cell proliferation rate. In addition, ANRIL is expected to modulate *in trans* the expression of genes distant from the *9p21* locus. This is evidenced by the differential expression of more than 200 genes involved in the maintenance of chromatin architecture, cellular proliferation, growth and apoptosis upon the overexpression of ANRIL sub-fragments in HeLa or in HEK293 cells. Expression changes were also observed for 20 genes, implicated in proliferation and apoptosis, upon ANRIL siRNA knockdown in vascular smooth muscle cells (VSMCs). Even though previous studies demonstrated the *trans*-regulatory activity of ANRIL, the molecular mechanisms involved need to be refined. Sor far, the coding and non-coding genes, which are directly contacted and regulated by ANRIL remain to be investigated in depth. Also, the mechanisms engaged by ANRIL to specifically associate with the genome remain to be deciphered.

In the past few years, an original system aimed at finely modulating gene expression by affecting the genomic recognition of the long non-coding RNA called ANRIL has been described (WO2022106623 ([3]). The authors have identified the genes outside the 9p21 locus directly regulated by ANRIL, and have showed that ANRIL contacts 3227 gene regions in human embryonic kidney cells (HEK293). Furthermore, the authors have identified 1477 and 1144 genes with increased or decreased expression respectively upon ANRIL knock-down. Then, the authors have focused on the 1477 genes whose expression was increased in the absence of ANRIL. They surprisingly identified 123 genes that were physically contacted and negatively regulated by ANRIL, and that correspond to ANRIL's primary gene targets. After extensive research, they identified a region within exon 8 of ANRIL (DBD-Ex8 for DNA Binding Domain-Exon8) capable of forming triplexes with 422 regions, and interestingly showed that 23 of them correspond to primary targets. It should be noted that among these 23 genes, several have been linked to pathologies related to ANRIL: cancers and vasculo/oculopathies (Alfeghaly et al., 2021 ([6])). These results were therefore consistent with ANRIL's regulatory activities on genes whose expression is deregulated in pathological conditions. The authors thus created a competitive molecule of the double-stranded DNA oligonucleotide type, designed to specifically block the formation of triplexes between genomic DNA and ANRIL, and named the latter ANRIL-TDO (ANRIL Triplex Decoy Oligonucleotide). Surprisingly, in its presence, ANRIL would be unable to bind to certain genomic loci via triplex formation and therefore unable to exercise its gene regulatory activity observed in pathological conditions. The highly innovative approach (targeted gene therapy) of this so-called epigenetic regulation involves using double-stranded DNA molecules (TDO) specifically designed to form stable triplexes with ANRIL. TDO can thus block ANRIL's ability to recognize its gene targets and therefore limit its activity, which operates inappropriately when overexpressed under pathological conditions. Therefore, it is important to mention that TDO relies on exon 8 of ANRIL as its activity support since it has been designed to associate with the latter through the formation of a triplex structure. In other terms, the core of TDO's activity lies in specifically targeting ANRIL's exon 8. Consequently, the ability to detect ANRIL's exon 8 from cancer cell lines and samples of tumor origin holds the promise of identifying tumors that are likely to respond to a TDO-based treatment.

So far, the standard method widely used for detecting RNA is named RT-qPCR (reverse transcription quantitative polymerase chain reaction) (Gadkar, Vija yJ, et Martin Filion. « New Developments in Quantitative Real-Time Polymerase Chain Reaction Technology ». Current Issues in Molecular Biology 16 (2014): 1-6 ([5])). However, this approach is not ideal for the precited goal, for the following main reasons:
1. Sequential properties of exon 8: Exon 8 is composed of repeated sequences, constituting approximately 70%. This characteristic makes it challenging to design primers that can specifically amplify exon 8 (Alfeghaly et al. « Implication of repeat insertion domains in the trans-activity of the long non-coding RNA ANRIL ». Nucleic Acids Research 49, n° 9 (21 mai 2021): 4954-70 ([6])). In pilot experiments attempting to detect exon 8 using RT-qPCR, the sequencing results of the generated amplicon did not yield successful outcomes (unpublished data).
2. Weak abundance of ANRIL: Long non-coding RNAs (LncRNAs), including ANRIL, are typically weakly expressed, with estimation at approximately 440 and 2200 copies of ANRIL per HEK293 and NCI-H146 cells, respectively (Alfeghaly et al., 2021 ([6]) and unpublished data). This low abundance necessitates at least 1 µg of total RNA as the starting material for RT-qPCR experiments. Achieving this quantity is challenging, especially when working with samples of tumor origin obtained, for example, through biopsy.
3. Cost: RT-qPCR can be relatively expensive, requiring specialized equipment, thereby contributing to the overall cost.

Together, these points demonstrate that RT-qPCR is not suitable for detecting exon 8 of ANRIL.

Thus, a need exists of alternative methods suitable for detecting exon 8 of ANRIL.

### Description of the invention

After extensive research, the Applicants have developed an original method, which he named HUNTER.

It involves a rapid and reliable method for detecting, at a lower cost, the presence of exon 8 of ANRIL.

The Applicants also provide a diagnostic kit based on the detection of exon 8, allowing the definition of a personalized therapeutic solution (-/+TDO) for patients suffering from cancer expressing ANRI L.

The method of the invention has many advantages:
- it has a very low detection threshold, using as little RNA as possible,
- it is faster than RT-qPCR: around 2h for the method of the invention compared with around 5h for RT-qPCR,
- it has a low cost as it does not necessitate a thermal cycler.

Accordingly, in a first aspect, the present invention provides a method of detecting the presence of exon 8 of ANRIL in a subject, comprising the following steps of:
1) Preparation of total RNA from a biological sample of the subject,
2) Conversion of RNA molecules into complementary DNA,
3) Loop-mediated isothermal amplification using trans-exonic oligonucleotides that specifically produces an amplicon containing exon 8 fragment,
4) Addition of Cas12a/RNA guide complexes, wherein RNA guide specifically targets exon 8-containing fragments by base pairing,
5) Addition of a reporter DNA probe, and
6) Detection of cleavage products,
Wherein detection of cleavage products reveals the presence of exon 8 of ANRIL.

According to the invention, "subject" refers to any mammal, e.g. humans and other primates. The subject may be a healthy subject, or a subject suffering from a pathology. For example, the pathology may be at least one among myocardial infarction, aneurysms, stenosis, cancers, eye diseases and type 2 diabetes.

"Biological sample" refers herein to any material that originates from the subject and that can be studied and analyzed in a laboratory. Taking the sample is not part of step 1). The sample may be for example selected among blood, plasma, serum, cells, tissue, urine, saliva, semen, feces/stool and bone marrow and sputum.

In step 1), the preparation of total RNA may be performed by any method known in the state of the art. It may be, for example, a method selected among Trizol^{™} method, Silica column method, for example using RNeasy mini kit (QIAGEN), alcohol precipitation method, affinity chromatography method, microfluidic method, agarose gel electrophoresis method, and density gradient ultracentrifugation method, this list not being exhaustive.

Step 2) of conversion of RNA, i.e. total RNA, molecules into complementary DNA (cDNA) may be carried out by any method known in the state of the art. It may be, for example, a method selected among Reverse Transcription (RT), Reverse Transcription Polymerase Chain Reaction (RT-PCR), Random Priming, Oligo(dT) Priming, and Target-Specific Priming, this list not being exhaustive. Preferably, the conversion is carried out by Reverse Transcription (RT).

Once complementary DNA is obtained, step 3) of Loop-mediated isothermal amplification (also called "LAMP") is performed, so as to obtain multicopy DNA. This step may be performed according to the method described by Soroka et al., 2021 (« Loop-Mediated Isothermal Amplification (LAMP): The Better Sibling of PCR? » Cells 10, no 8 (29 juillet 2021): 1931 ([7]). If necessary, routine adjustments can be made to this method, according to the general knowledge of those skilled in the art. This technique enables the multiplication of target copies for detection, i.e. exon 8, to reach a critical detection threshold. Advantageously, it is highly specific, requiring a combination of an optimum of 4 to 6 oligonucleotides designed to hybridize several distinct locations within the target cDNA produced in step 2) (as opposed to only 2 for standard PCR). Furthermore, this technique drastically increases the quantities of amplified DNA of interest (up to 1 billion copies in 1 hour, compared to 1 million for standard PCR, due to the generation of multiple initiation sites during the reaction). Also, this technique does not require sophisticated equipment like a thermocycler. The reaction is carried out at a constant temperature of 65°C due to the intrinsic properties of the Bst DNA polymerase used in the approach described by Soroka et al. ([7]).

According to the invention, LAMP is carried out with exonic and trans-exonic oligonucleotides. "Trans-exonic oligonucleotides" refers herein to oligonucleotides, also called "primers", designed to amplify regions of a gene, that span multiple exons and/or hybridize to exon-exon junctions. In the present case, these multiple exons mean exon 6, exon 7 and exon 8.

Based on extensive research, it has been determined that using trans-exonic primers, especially at 6exon-7exon and 7exon-8exon junctions, is crucial for amplifying specific regions, such as exons 6, 7 and 8. These primers enable precise hybridization outside any repetitive genomic regions and ensure that amplification targets exclusively cDNA containing exons 6, 7 and 8, as they do not bind to intronic sequences present in genomic DNA. This strategic placement provides specific amplification and reduces false positives attributed to genomic DNA contamination in RNA samples, eliminating the necessity for additional DNase treatment. The major advantage of using primers at exon-exon junctions is that it further ensures the specificity of amplification, enhancing the accuracy and reliability of the exon 8 detection. It is important to note that this is the first time that trans-exonic oligonucleotides are used in a LAMP amplification.

Advantageously, the trans-exonic oligonucleotides may be at least one chosen among:
- O1: TCTATTTGGAATGTATCTAACTCC (SEQ ID NO: 1),
- O2: TTCATGCTTTGGATATCAGTG (SEQ ID NO: 2),
- O3:
   AGTTCAAGACCAGTCTGGCCGAAACCATCAGAGGTAACAGTA (SEQ ID NO: 3),
- O4:
   CAAAGTGCTGGGATTACAGGTGAAGGCGTTCATTGGAGAG (SEQ ID NO: 4),
- O5: AACATGGTGAAACCCCGTCTC (SEQ ID NO: 5),
- O6: ACCACACCCGGCGGATA (SEQ ID NO: 6).

Advantageously, a combination of 2 to 6, preferably 4 to 6, of these trans-exonic oligonucleotides may be used. The oligonucleotides may be at least one combination chosen among:
- a mix of O3 and O4,
- a mix of O1 and O2,
- a mix of O5 and O6.

Any combinations of these mixes are possible, according to the following list:
- O3 and O4 and O1 and O2
- O3 and O4 and O5 and O6,
- O1 and O2 and O5 and O6
- O1 and O2 and O3 and O4 and O5 and O6.

As mentioned above, trans-exonic oligonucleotides can specifically produce an amplicon containing exon 8 fragment. This exon 8 fragment is about the 2.7% of the sequence of exon 8 (i.e the 19-nts of the 5' extremity of exon 8). The produced amplicon contains, in addition to exon 8 fragment, exon 6 fragment and exon 7 fragment, which are from 5' side of exon 8 fragment. According to the invention, "specifically" means that the method produces an amplicon containing exon 8 fragment at a level of at least 95%, for example 96%, 97%, 98%, 99% or about 100%, of the total number of amplicons formed.

Regarding step 4) of the method, CRISPR/Cas12a system used may be performed according to the method described by Chen et al. ([8]), Broughton et al. ([9]), Sun et al. ([10]) or Paul and Montoya ([11]), also known as "DETECTR method". If necessary, routine adjustments can be made to this method, according to the general knowledge of those skilled in the art. Cas12a and RNA guide may be added separately, and they can form complexes in the reaction medium. As already described in the state of the art, the CRISPR/Cas12a system requires the involvement of two elements: an endonuclease called Cas12a and a guide RNA, also called herein "Cas12a/RNA guide complexes". The latter results from the fusion of two functional RNAs: the crRNA, which facilitates the recognition of a targeted DNA region by being for example about 20 nucleotides complementary to the target, and the tracrRNA (5'-UAAUUUCUACUAAGUGUAGAU-3') (SEQ ID NO: 11), which is responsible for Cas12a association. The formed ribonucleoprotein (RNP) is capable of specifically binding to the 20-nucleotides targeted DNA region and catalyzing the degradation of the targeted region upstream of a Protospacer Adjacent Motif (PAM) sequence of type 5'-TTTN-3'. In addition to this targeted degradation, the Cas12a has an additional activity termed collateral. Once activated, Cas12a also degrades any nearby DNA fragment, regardless of its sequence. In the method of the invention, this "nonspecific" activity is used to cleave a reporter DNA fragment, allowing to indirectly assess the presence of the RNA of interest, i.e. the exon 8 fragment.

In Cas12a/RNA guide complex, i.e., Cas12a complexed with RNA guide, said RNA guide may have any sequence that specifically targets exon 8-containing fragment by base pairing. In other words, RNA guide may recognize exon7-exon8 junction and hybridize with it. Advantageously, the RNA guide hybridizes on the exon7-exon8 junction which provides specific recognition of the amplicons of interest and reduces false positives attributed to genomic DNA contamination in RNA samples.
For example, RNA guide may comprise the sequence, or consist of the sequence: 5'-UAAUUUCUACUAAGUGUAGAUACAGUCUCUCCAAUGAACGCC-3' (SEQ ID NO: 7). The RNA guide may comprise, on one side or on both sides, sequences to provide stability to the ends of the guide RNA formats, to protect them against degradation by environmental and intracellular nucleases.

The reporter probe may be added in the reaction medium at the same time as the guide RNA and Cas12a. Alternatively, it may be added after the formation of the Cas12a/RNA guide complexes in the reaction medium. The reporter DNA probe may be any DNA sequence. Advantageously, the probe may have a fluorophore at one side, in order to facilitate its detection, and eventually a quencher at the other side, in order to help detecting the probe or reading the results, as is known in the art. For example, the reporter DNA probe may be coupled 5' to 6-Carboxyfluorescein (F) and 3' to biotin (B). For example, the DNA probe may have a sequence chosen among the sequence TTATTATT, TTATT and/or TTATTATTATT (SEQ ID NO: 8).

Once the guide RNA forms a complex with Cas12a and directs it to the target sequence, Cas12a undergoes a conformational change that activates its endonuclease activity. When Cas12a is activated and begins cleaving the target DNA, it may also cleave the nearby fluorescent probe because of the Cas12a collateral activity. Then, cleavage of the probe releases the fluorescent reporter molecule, commonly a fluorescent reporter molecule, resulting in a detectable and possibly measurable change in fluorescence signal. Detection may be carried out by means commonly used in the state of the art, for example with anti-fluorescein antibodies, especially on strip, fluorescence spectroscopy, fluorescence plate readers, flow cytometry, gel electrophoresis, this not being exhaustive.

Optionally, after step 6), a step 7) may be included to quantify the abundance of exon 8 of ANRIL by measuring the intensity of the band that appears on the strip. This may be performed using conventional devices such as densitometer, portable strip test reader, colorimeter or scanner.

All definitions and explanations of technical features or terms given in relation with the method of detection of the invention apply to the following other objects of the invention.

Another object of the invention relates to a method for predicting in vitro the efficacy of a therapy targeting exon 8 of ANRIL in a subject, comprising the steps of:
- detecting the presence of exon 8 of ANRIL in a biological sample of a subject with a method of detecting the presence of exon 8 of ANRIL as defined above,
   wherein:
- a presence of exon 8 of ANRIL is predictive of the subject's response to the therapy targeting exon 8 of ANRIL, and,
- an absence of exon 8 of ANRIL is predictive of the subject's absence of response to the therapy targeting exon 8 of ANRIL.

Advantageously, the therapy may comprise an isolated double stranded DNA polynucleotide that forms triplex with sequence
5'-GGUGGCAGCAAGAGAAAAAUGAGGAAGAAGCAAAAGCGGAAA-3' (SEQ ID NO: 9) of the long non-coding RNA ANRIL. Sequence SEQ ID NO: 9 is a region within exon 8 (the full sequence of which being represented as SEQ ID NO: 10) of ANRIL (DBD-Ex8 for DNA Binding Domain-Exon 8) previously identified by the Applicants.

As mentioned previously, the subject may be suffering from a pathology chosen among myocardial infarction, aneurysms, stenosis, cancers, eye diseases or type 2 diabetes.

Another object of the invention relates to a kit for implementing the method as defined above, comprising the following elements:
- trans-exonic oligonucleotides for specific exon 8-containing fragment amplification,
- RNA guide specifically targeting exon 8-containing fragment by base pairing,
- Reagents for RT-LAMP amplification and Cas12a digestion reactions,
- a reporter DNA probe, and
- a mean for the detection of cleavage products.

Another object of the invention relates to a set of oligonucleotides having at least one sequence chosen among:
- O1: TCTATTTGGAATGTATCTAACTCC (SEQ ID NO: 1),
- O2: TTCATGCTTTGGATATCAGTG (SEQ ID NO: 2),
- O3:
   AGTTCAAGACCAGTCTGGCCGAAACCATCAGAGGTAACAGTA (SEQ ID NO: 3),
- O4:
   CAAAGTGCTGGGATTACAGGTGAAGGCGTTCATTGGAGAG (SEQ ID NO: 4),
- O5: AACATGGTGAAACCCCGTCTC (SEQ ID NO: 5), or
- O6: ACCACACCCGGCGGATA (SEQ ID NO: 6).

Advantageously, the set of oligonucleotides may comprise a mix of oligonucleotides chosen among:
- a mix of O3 and O4,
- a mix of O1 and O2, and
- a mix of O5 and O6.

Another object of the invention relates to a use of a set of oligonucleotides as defined above, or of a kit as defined above, for predicting the efficacy of a therapy targeting exon 8 of ANRIL in a subject. Indeed, as explained above, the ability to detect ANRIL's exon 8 from cancer cell lines and samples of tumor origin holds the promise of identifying tumors that are likely to respond to a TDO-based treatment.

This invention is further illustrated by the following examples with regard to the annexed drawings that should not be construed as limiting.

### Brief description of the figures

- Figure 1: represents the position on exons 6, 7 and 8 of the trans-exonic primers used for the detection of ANRIL isoforms containing exon 8.
- Figure 2: represents the lateral flow strip assay readout showing the results of the detection method. Right hand column: method performed on HCT116 cells, showing a band on control and a band at ANRIL exon 8. Left hand column: control method performed with no RNA only showing the control band.

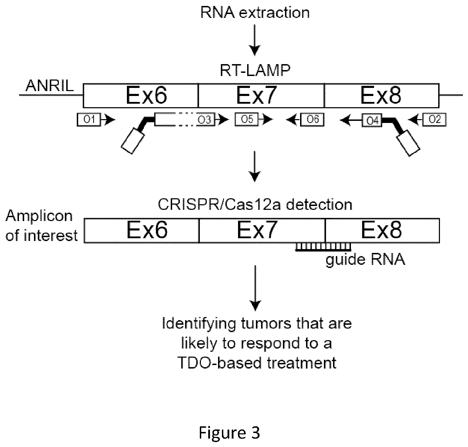

- Figure 3: represents a diagram of implementation of the method for in vitro predicting the efficacy of a therapy targeting exon 8 of ANRIL in a subject.

### Examples

### Example 1: Implementation of the method of detecting the presence of exon 8 of ANRIL in a subject

### 1. Primer sequences

### 1.1. Primer sequences used for the detection of ANRI L isoforms containing exon 8 (Ex6-7-8)

Primer sequences (Merck Sigma Aldrich) used are the following:
O1: TCTATTTGGAATGTATCTAACTCC (SEQ ID NO: 1), which spans on exon 6
O2: TTCATGCTTTGGATATCAGTG (SEQ ID NO: 2), which spans on exon 8
O3:
   AGTTCAAGACCAGTCTGGCCGAAACCATCAGAGGTAACAGTA (SEQ ID NO: 3), which spans on exons 6 and 7
O4: CAAAGTGCTGGGATTACAGGTGAAGGCGTTCATTGGAGAG (SEQ ID NO: 4), which spans on exon 8
O5: AACATGGTGAAACCCCGTCTC (SEQ ID NO: 5). This primer has been specifically designed to hybridize within the amplicon generated by primer O3. Primer O3 is engineered to produce an amplicon that forms a hairpin structure as its 5' extremity, with a single-stranded loop. This loop can serve as a hybridization site for another primer, such as O5. By base-pairing with the loop, O5 creates an additional initiation site for amplification, enhancing the overall efficiency of the LAMP reaction.
O6: ACCACACCCGGCGGATA (SEQ ID NO : 6). This primer has been specifically designed to hybridize within the amplicon generated by primer O4. Primer O4 is engineered to produce an amplicon that forms a hairpin structure as its 3' extremity, with a single-stranded loop. This loop can serve as a hybridization site for another primer, such as O6. By base-pairing with the loop, O6 creates an additional initiation site for amplification, enhancing the overall efficiency of the LAMP reaction.

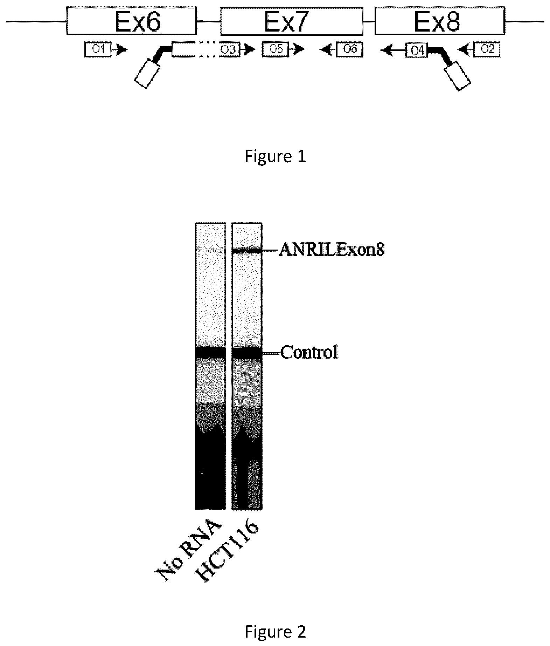

### The positions of these primers on exons 6, 7 and 8 appear in Figure 1. 1.2. Guide RNA sequence for cleavage by Cas12a (IDT)

CrRNA ex6-7-8 is as follows:

/AltR1/UAAUUUCUACUAAGUGUAGAUACAGUCUCUCCAAUGAA
CGCC/AltR2/(SEQ ID NO: 7)

### 1.3 Probe sequence (IDT)

### OProbe: 5'/6-FAM/TTATTATT/Bio/-3', HPLC purified

### 2. LAMP reaction

| **Components of the reaction mix** | **Detection of ANRIL exon 8** | **No template control** |
|---|---|---|
| SuperScript IV RT-LAMP Master Mix (NEB Biolabs) | 12.5 µL | 12.5 µL |
| 40 µM primers O3 and O4 Mix | 1 µL | 1 µL |
| 10 µM primers O1 and O2 Mix | 0.5 µL | 0.5 µL |
| 10 µM primers O5 and O6 Mix | 1 µL | 1 µL |
| Total RNA (10 ng/µL) | 1 µL | |
| Nuclease-free water | 9 µL | 10 µL |

| | | |
|---|---|---|
| Incubate for 60 min at 65°C Incubate for 2 min at 95°C | | |

### 3. Cas12a-mediated cleavage reaction

**Complex formation**

| | Stock | Final | Dilution factor | 18 µL |
|---|---|---|---|---|
| Cas12a | 5 µM | 250 nM | 20 | 0.9 µL |
| guide RNA | 6.25 µM | 62.5 nM | 100 | 0.18 µL |
| 10x NEB2.1 | 10x | 1x | 10 | 1.8 µL |
| H2O | | | | 15 µL |

| | | | | |
|---|---|---|---|---|
| Incubate for 10 min at 37°C | | | | |

**Add Probe**

| | Stock | Final | Dilution factor | 18 µL |
|---|---|---|---|---|
| reporter DNA Probe Oprobe A | 50 µM | 500 nM | 100 | 0.18 µL |

Prepare the following mixture
1 µL LAMP reaction (diluted 1/16)
18 µL Cas12a-RNA guide complex + OProbe
81 µL 1X NEB2.1 buffer (Biolabs)
Incubate for 20 min at 37°C

### 4. Detection (Milenia Hybridetect)

Mix 50 µL of cleavage reaction with 50 µL of Hybridetect (Milenia Biotec) Assay Buffer (1:1) and incubate 5 min at room temperature (RT), which corresponds to about 20°C.

Place the HybriDetect Dipstick with the sample application into the solution mix and incubate 5 min at RT in an upright position.

Then remove dipsticks from the assay solution and interpret test result immediately.

### 5. Results obtained by applying HUNTER on TDO-sensitive HCT116 cells

As a proof of principle, we tested the detection method of the invention on HCT116 cells, assuming that, since they respond to TDO treatment (see WO2022106623 ([3])), they must express exon 8-ANRIL isoforms. In this scenario, a positive result is anticipated. Interestingly, the results shown in Figure 2 are coherent with this assumption, as a band corresponding to the antibody line is observed on the strip, which is not the case for the negative control.

In conclusion, these results demonstrate that the detection of exon 8 of ANRIL allows the prediction of the activity of novel potential treatments against cancer, i.e., TDO.

### Reference List

1. Rajagopal and Feigon: "Triple-Strand Formation in the Homopurine:homopyrimidine DNA Oligonucleotides d(G-A)4 and d(T-C)4." Nature 339, no. 6226 (June 22, 1989): 637-40.
2. Maldonado et al.: "Purine- and Pyrimidine-Triple-Helix-Forming Oligonucleotides Recognize Qualitatively Different Target Sites at the Ribosomal DNA Locus." RNA (New York, N.Y.) 24, no. 3 (2018): 371-80.
3. WO 2022/106623.
4. Crinelli et al.: "Locked Nucleic Acids (LNA): Versatile Tools for Designing Oligonucleotide Decoys with High Stability and Affinity." Current Drug Targets 5, no. 8 (November 2004): 745-52.
5. Gadkar et Filion. « New Developments in Quantitative Real-Time Polymerase Chain Reaction Technology ». Current Issues in Molecular Biology 16 (2014): 1-6.
6. Alfeghaly et al.: « Implication of repeat insertion domains in the trans-activity of the long non-coding RNA ANRIL». Nucleic Acids Research 49, n° 9 (21 mai 2021): 4954-70.
7. Soroka et al.: « Loop-Mediated Isothermal Amplification (LAMP): The Better Sibling of PCR? » Cells 10, no 8 (29 juillet 2021): 1931.
8. Chen et al.: « CRISPR-Cas12a Target Binding Unleashes Indiscriminate Single-Stranded DNase Activity ». Science (New York, N.Y.) 360, no 6387 (27 avril 2018): 436 39.
9. Broughton et al. « CRISPR-Cas12-Based Detection of SARS-CoV-2 ». Nature Biotechnology 38, n° 7 (juillet 2020): 870-74.
10. Sun et al.: « One-Tube SARS-CoV-2 Detection Platform Based on RT-RPA and CRISPR/Cas12a ». Journal of Translational Medicine 19, n° 1 (16 février 2021): 74.
11. Paul, Bijoya, et Guillermo Montoya: « CRISPR-Cas12a: Functional Overview and Applications ». Biomedical Journal 43, n° 1 (février 2020): 8-17.

## Claims

1. Method of detecting the presence of exon 8 of ANRIL (Antisense Non-coding RNA in the INK4 Locus) in a subject, comprising the following steps of:
1) Preparation of total RNA from a biological sample of the subject,
2) Conversion of RNA molecules into complementary DNA,
3) Loop-mediated isothermal amplification using trans-exonic oligonucleotides that specifically produces an amplicon containing exon 8 fragment,
4) Addition of Cas12a/RNA guide complexes, wherein RNA guide specifically targets exon 8-containing fragment by base pairing,
5) Addition of a reporter DNA probe, and
6) Detection of cleavage products,
Wherein detection of cleavage products reveals the presence of exon 8 of ANRIL.

2. Method according to claim 1, wherein said trans-exonic oligonucleotides that specifically produce an amplicon containing exon 8 fragment are at least one chosen among:
- O1: TCTATTTGGAATGTATCTAACTCC (SEQ ID NO: 1),
- O2: TTCATGCTTTGGATATCAGTG (SEQ ID NO: 2),
- O3:
AGTTCAAGACCAGTCTGGCCGAAACCATCAGAGGTAACAGTA (SEQ ID NO: 3),
O4: CAAAGTGCTGGGATTACAGGTGAAGGCGTTCATTGGAGAG (SEQ ID NO: 4),
O5: AACATGGTGAAACCCCGTCTC (SEQ ID NO: 5),
O6: ACCACACCCGGCGGATA (SEQ ID NO: 6).

3. Method according to claim 2, wherein said oligonucleotides are at least one chosen among:
- a mix of O3 (SEQ ID NO: 3) and O4 (SEQ ID NO: 4),
- a mix of O1 (SEQ ID NO: 1) and O2 (SEQ ID NO: 2),
- a mix of O5 (SEQ ID NO: 5) and O6 (SEQ ID NO: 6).

4. Method according to any one of the preceding claims, wherein said RNA guide comprises the sequence
UAAUUUCUACUAAGUGUAGAUACAGUCUCUCCAAUGAACGCC (SEQ ID NO: 7).

5. Method according to any one of the preceding claims, wherein said reporter DNA probe is coupled 5' to 6-Carboxyfluorescein (F) and 3' to biotin (B).

6. Method according to claim 5, wherein said reporter DNA probe is TTATTATT, TTATT and/or TTATTATTATT (SEQ ID NO: 8).

7. Method according to any one of the preceding claims, wherein detection of cleavage products is carried out by anti-fluorescein antibodies.

8. Method for in vitro predicting the efficacy of a therapy targeting exon 8 of ANRIL in a subject, comprising the steps of:
- detecting the presence of exon 8 of ANRIL in a biological sample of a subject with a method as defined in any one of claims 1 to 7,
wherein:
- a presence of exon 8 of ANRIL is predictive of the subject's response to the therapy targeting exon 8 of ANRIL, and,
- an absence of exon 8 of ANRIL is predictive of the subject's absence of response to the therapy targeting exon 8 of ANRIL.

9. Method according to claim 8, wherein said therapy comprises an isolated double stranded DNA polynucleotide that forms triplex with sequence
GGUGGCAGCAAGAGAAAAAUGAGGAAGAAGCAAAAGCGGAAA (SEQ ID NO: 9) of the long non-coding RNA ANRIL.

10. Method according to claim 8 or 9, wherein the subject is suffering from a pathology chosen among myocardial infarction, aneurysms, stenosis, cancers, eye diseases or type 2 diabetes.

11. Kit for implementing the method as defined in any one of claims 1 to 7, comprising the following elements:
- trans-exonic oligonucleotides for specific exon 8-containing fragment amplification,
- RNA guide specifically targeting exon8-containing fragment by base pairing,
- Reagents for RT-LAMP amplification and Cas12a digestion reactions,
- a reporter DNA probe, and
- a mean for the detection of cleavage products.

12. Set of oligonucleotides having at least one sequence chosen among:
- O1: TCTATTTGGAATGTATCTAACTCC (SEQ ID NO: 1),
- O2: TTCATGCTTTGGATATCAGTG (SEQ ID NO: 2),
- O3:
AGTTCAAGACCAGTCTGGCCGAAACCATCAGAGGTAACAGTA (SEQ ID NO: 3),
O4: CAAAGTGCTGGGATTACAGGTGAAGGCGTTCATTGGAGAG (SEQ ID NO: 4),
O5: AACATGGTGAAACCCCGTCTC (SEQ ID NO: 5), or
O6: ACCACACCCGGCGGATA (SEQ ID NO: 6).

13. Set of oligonucleotides according to claim 12, chosen among:
- a mix of O3 (SEQ ID NO: 3) and O4 (SEQ ID NO: 4),
- a mix of O1 (SEQ ID NO: 1) and O2 (SEQ ID NO: 2), and
- a mix of O5 (SEQ ID NO: 5) and O6 (SEQ ID NO: 6).

14. Use of a kit as defined in claim 11 or of a set of oligonucleotides according to claim 9 or 10, for predicting the efficacy of a therapy targeting exon 8 of ANRIL in a subject.
